# EUROPEAN PATENT SPECIFICATION

(11) **EP 0 322 533 B1**
(45) Date of publication and mention of the grant of the patent: **24.05.1995**
(21) Application number: 88117742.2
(22) Date of filing: 25.10.1988
(51) Int. Cl.: C12N 15/31, A61K 39/10

(54) **Bordetella pertussis toxin with altered toxicity**
Bordetella pertussis-Toxin mit veränderter Toxizität
Toxine de bordetella pertussis ayant une toxicité modifiée

(30) Priority: 02.11.1987 IT 2248187
(43) Date of publication of application: 05.07.1989
(73) Proprietor: SCLAVO S.p.A., I-53100 Siena (IT)
(72) Inventor: Pizza, Mariagrazia, I-53100 Siena (IT); Rappuoli, Rino, I-53010 Quercegrossa Siena (IT); Bartoloni, Antonella, I-53035 Monteriggioni Siena (IT)
(74) Representative: Hallybone, Huw George

(56) References cited:
- EP-A- 0 232 229
- EP-A- 0 275 689
- INFECTION AND IMMUNITY, vol. 55, no. 11, November 1987, pages 2546-2553,American Society for Microbiology; C. LOCHT et al.: "Activities of complete andtruncated forms of pertussis toxin subunits S1 and S2 synthesized byEscherichia coli"
- SCIENCE, vol. 232, 6th June 1986, pages 1258-1264; C. LOCHT et al.: "Pertussistoxin gene: nucleotide sequence and genetic organization"
- PROCEEDINGS OF THE NATIONAL ACADEMY OF SCIENCES USA, vol. 83, July 1986, pages4631-4635; A. NICOSIA et al.: "Cloning and sequencing of the pertussis toxingenes: Operon structure and gene duplication"
- INFECTION AND IMMUNITY, vol. 55, no. 4, April 1987, pages 963-967, AmericanSociety for Microbiology; A. NICOSIA et al.: "Expression and immunologicalproperties of the five subunits of pertussis toxin"
- INFECTION AND IMMUNITY, vol. 55, no. 5, May 1987, pages 1321-1323, AmericanSociety for Microbiology; J.T. BARBIERI et al.: "Expression of the S-1catalytic subunit of pertussis toxin in Escherichia coli"
- INFECTION AND IMMUNITY, vol. 56, no. 8, August 1988, pages 1934-1941, AmericanSociety for Microbiology; J.T. BARBIERI et al.: "AFP-ribosyltransferasemutations in the catalytic S-1 subunit of pertussis toxin"
- SCIENCE, vol. 242, no. 4875, 1988, pages 72-74; W.N. BURNETTE et al.:"Pertussis toxin S1 mutant with reduced enzyme activity and a conserved protective epitope"
- EP-A1-0 306 318

## Description

The present invention relates to immunologically active polypeptides with no or reduced toxicity useful for the production of an antipertussis vaccine.

The invention also relates to a method for the preparation of said polypeptides and to an antipertussis vaccine comprising a therapeutically effective amount of at least one of said polypeptides.

Pertussis is a respiratory system disease caused by *Bordetella pertussis (B. pertussis)*, a bacillus, the transmission of which occurs during the catarrhal and convulsive phase of the disease from a sick person to a healthy predisposed individual through the respiratory system.

A vaccine effective against said disease is particularly desirable since pertussis may cause convulsions, cerebral damage and, sometimes, death, principally in young children and in newborns lacking maternal antipertussis antibodies.

At present, an antipertussis vaccine is used which comprises virulent bacteria killed with merthiolate and treated at 56°C which, even if it confers permanent protection, is not completely satisfactory, either because of the presence of undesired side effects or because of numerous problems arising from the preparation and purification thereof.

This results in a need for an antipertussis vaccine lacking of the aforementioned drawbacks.

It is known that *B. pertussis* ha*s, per se,* no virulence and that its toxicity is correlated with the synthesis, during the virulent phase, of such substances as: hemolysin (Hls), adenylcyclase (Adc), dermonecrotic toxin (Dnc), filamentary haemagglutinin (Fha) and pertussis toxin (PT). The latter, in particular, represents not only the major virulence factor produced by *B. pertussis* (Weiss A. *et al*. (1983) Infect, Immun. 42, 333-341; Weiss A. *et al*. (1984) J. Inf. Dis. 150, 219-222) but also one of the major protective antigens against infections caused by said bacterium.

Anti-PT antibodies have been found in individuals immunized by the cellular vaccine (Ashworth L.A.E. *et al*. (1983) Lancet. Oct. 878-881) and protective immunity has been demonstrated in mice infected, via aereosol or intracerebrally, using formaldehyde-detoxified PT (Sato Y. *et al*. (1983) Inf. and Imm. 41, 313) . Even if PT represents an essential component in the preparation of new antipertussis vaccines, its use is limited by the numerous drawbacks arising from its toxicity.

PT induces undesirable pathophysiological effects such as: lymphocytosis, histamine sensitivity, hypoglycemia, insensitivity to the hyperglycemic effect of epinephrine and activation of the Islets of Langerhans.

Furthermore, it has been found that PT present in the vaccine now employed is the principal cause of such side effects as: fever, pomphus, neurological alteration and death which have led, in recent years, to drastically reduced use of the vaccine with consequent new outbreaks of pertussis cases.

PT detoxification treatment by means of formaldehyde, although providing an immunogenic protein without toxicity (Sato *et al*. reference reported above) , presents some drawbacks arising from the fact that said protein is not obtainable in pure, reproducible and stable form.

According to the present invention, polypeptides have now been found which are able to overcome the prior art drawbacks and are obtainable in pure form by means of a simple and economically feasible method. One object of the present invention, therefore, is the provision of immunologically active polypeptides with no or reduced toxicity useful for the preparation of an antipertussis vaccine.

A further object of the present invention consists of a method for the preparation of said polypeptides.

Another object of the present invention is a vaccine comprising a therapeutically effective amount of at least one of said polypeptides.

Further objects of the present invention will become apparent from a reading of the following description and examples.

PT is a protein comprising five different subunits, the toxicity of which is due to ADP-ribosylation of proteins which bind GTP involved in the transmission of messages through eukaryotic cell membranes.

PT comprises two domains with different functionality: the A domain comprising the S1 subunit and the B domain comprising S2, S3, S4 and S5 subunits organised in two dimers, D1 (S2+S4) and D2 (S3+S4) linked to each other by an S5 subunit.

The A domain represents the enzymatically active and therefore toxic part of PT, whereas the B domain binds eukaryotic cell membrane receptors and allows the introduction of the S1 subunit therein.

In copending European patent application EP-A-0 232 229 the cloning, sequencing and expression of the genes which code for said subunits have been described and claimed and it has been shown that said genes are grouped in a sole operon.

Furthermore, the ADP-ribosylation activity of the S1 subunit has been determined, by cultivating a micro-organism transformed with the hybrid plasmid PTE225, and it has been found that said subunit possesses an enzymatic activity comparable to that of PT.

According to the present invention, there is provided a protein having the immunological and protective properties of the PT, but with no or reduced toxicity.

The positions and nature of the fundamental amino acids for the enzymatic activity of the protein have been identified. In particular, the following positions and amino acids have been found:
tyrosine (8), arginine (9), phenylalanine (50), threonine (53) , glutamic acid (129), glycine (121) , alanine (124), aspartic acid (109), glycine (99), arginine (135), threonine (159) and tyrosine (111).

The substitution of one of more of said amino acids with a different amino acid provides a protein with altered toxicity.

According to the present invention, polypeptides have been synthesized containing the S1 subunit of the modified PT by means of direct mutagenesis substituting, in one or more positions of the S1 region between amino acids 1-180, one amino acid with another capable of destroying or reducing its enzymatic activity without altering the immunological properties thereof.

In particular, polypeptides have been synthesized containing the S1 subunit of PT modified by substituting:
- the tyrosine in position 8 and arginine in position 9 with aspartic acid and glycine respectively;
- the phenylalanine in position 50 and the threonine in position 53 with glutamic acid and isoleucine respectively;
- the glutamic acid in position 129 with glycine;
- the glycine in position 121 with glutamic acid;
- the alanine in position 124 with aspartic acid;
- the aspartic acid in position 109 and the alanine in position 124 with glycine and aspartic acid respectively;
- the glycine in position 99 with glutamic acid;
- the aspartic acid in position 109 with glycine;
- the arginine in position 135 with glutamic acid;
- the threonine in position 159 with lysine;
- the tyrosine in position 111 with glycine and insertion of Asp Thr Gly and Gly amino acids in position 113.

In particular, the polypeptides according to the present invention have been prepared by a method which comprises:
a) modifying, by means of direct mutagenesis, the gene which codes for the S1 subunit of the pertussis toxin substituting, in one or more sites of the DNA molecule, the base sequence which codes for a predetermined amino acid with a base sequence which codes for the amino acid of interest;
b) constructing a hybrid plasmid linking a cloning vector with the DNA fragment containing the modified S1;
c) transforming a host microorganism with a hybrid plasmid obtained as reported in b);
d) cultivating in a suitable culture medium, in the presence of carbon, nitrogen and mineral salts sources, a transformed microorganism; and then,
e) recovering the polypeptide containing the modified S1 subunit from the culture medium or from the cells.

According to the present invention, to identify the S1 amino acid region correlated with the enzymatic activity of the protein, the gene which codes for S1 has been treated with restriction enzymes that cut in different sites and the DNA fragments so obtained, lacking different length 3' or 5' terminal sequences, have been cloned in an expression plasmid according to generally known techniques. The vectors containing the DNA fragments with the deleted sequences have been then employed to transform *Escherichia coli (E. coli)* cells.

Positive transformants, obtained by screening the cells on a selective medium, have been cultivated in a suitable culture medium at temperatures between 30°C and 40°C for a period of from 20 minutes to 5 hours.

At the end of said period, the cells have been recovered from the culture medium and lysed by means of lysozyme treatment and sonication.

The proteins so extracted have been analysed, to determine enzymatic activity.

The ADP-ribosylation activity of said proteins was tested according to the method described by Manning *et al*. (1984) (J. Biol. Chem. 259, 749-756).

The results obtained, listed in Table I of Example 2, show that S1 sequences following the amino acid in position 179, are not necessary for the ADP-ribosylation activity, unlike the first ten amino acids.

The enzymatically active region of the S1 subunit, therefore, is located between the amino acids 1 and 180. In accordance with the present invention, the identification of the active sites present in said region has been performed and at least one of said sites has been modified.

In practice, the gene coding for S1 has been isolated from the PTE 255 plasmid, the construction of which is reported in the European patent application EP-A-0 232 229, by means of digestion with the restriction enzymes EcoRI and HindIII.

A 600 base pair DNA fragment, comprising the gene coding for S1, has been separated from the digestion mixture by means of gel electrophoresis and, after electro-elution, has been modified by direct mutagenesis which produces *in vitro* mutations at determined sites of the DNA molecule. This permits the effect of said mutation to be tested *in vitro* or *in vivo*.

Substitution of the desired base is achieved in one of the following ways:
- by incorporating base analogues in DNA sites;
- by incorporating nucleotides incorrectly;
- by introducing a mutation during the synthesis *in vivo* of oligonucleotides with definite sequences; or
- by using specific chemical mutagenic agents, such as sodium bisulphite, which react with the DNA bases.

According to the present invention, the gene coding for S1 has been modified by using synthetic oligonucleotides with definite sequences operating according to the method described by Zoller M.J. *et al*. (DNA 3:479-488, (1984)).

In practice, the 600 bp DNA fragment has been cloned in a vector which allows the isolation of single-stranded DNA.

To this end, suitable vectors may be selected from Bluescript SK (Stratagene San Diego, CA), pEMBL (Dente *et al*. Nucleic Acids Research 11, 1645-1655 (1983)) or M13 phages (Viera and Messing (1982) Gene, 19, 263)).

According to the present invention, the commercially available Bluescript SK vector was employed.

Said vector was treated with suitable restriction enzymes and then linked to the 600 bp DNA fragment in a ligase mixture in the presence of the T4 DNA ligase enzyme.

The mixture was then employed to transform *E. coli* cells and the transformants were successively selected on a culture medium including ampicillin.

The positive clones, containing the hybrid plasmids comprising the vector and the 600 bp DNA fragment, were suspended in a liquid medium in the presence of phages and maintained at a temperature of from 30°C to 40°C for a period of from 2 to 10 hours.

At the end of said period, the phages were precipitated, separated from the solution by centrifugation, resuspended in a pH 7.5 buffer, extracted with water-ethyl ether saturated phenol and then extracted with ethanol and ammonium acetate in order to precipitate the single-stranded DNA.

Aliquots of said DNA were employed to modify the S1 gene by direct mutagenesis. To this end, oligonucleotides of about 20 nucleotides were synthesized in which the bases which code for one or more amino acids present in determined sites of the 1-180 S1 region were substituted with others which code for a different amino acid. In particular, oligonucleotides were synthesized which allow to prepare the following mutants of the gene coding for S1:
- 41:: Tyrosine 8 and arginine 9 were substituted with aspartic acid and glycine respectively using the primer GTCATAGCCGTCTACGGT.
The corresponding gene was modified in this way:
620-CGCCACCGTATACCGCTATGACTCCCGCCCG-650
620-CGCCACCGTAGACGGCTATGACTCCCGCCCG-650
- 22:: Phenylalanine 50 and threonine 53 were substituted with glutamic acid and isoleucine respectively using the primer TGGAGACGTCAGCGCTGT.
The corresponding gene was modified in this way:
The sequence 750-AGCGCTTTCGTCTCCACCAGC-770 was changed into etc.
- 25:: Glycine 99 was substituted with glutamic acid using the primer CTGGCGGCTTCGTAGAAA.
The corresponding gene was modified in this way:
the sequence 910-TACGGCGCCGC-920 was changed into 910-TACGAAGCCGC-920.
- 17:: Aspartic acid 109 was substituted with glycine using the primer CTGGTAGGTGTCCAGCGCGCC.
The corresponding gene was modified in this way:
the sequence 930-GTCGACACTTA-940 was changed into 930-GTCGGCACTTA-940.
- 27:: Glycine 121 was substituted with glutamic acid using the primer GCCAGCGCTTCGGCGAGG.
The corresponding gene was modified in this way:
the sequence 956-GCCGGCGCGCT-966 was changed into 956-GCCGAAGCGCT-966.
- 16:: Alanine 124 was substituted with aspartic acid using the primer GCCATAAGTGCCGACGTATTC.
The corresponding gene was modified in this way:
the sequence 976-TGGCCACCTAC-984 was changed into 976-TGGACACCTAC-986.
- 1716:: contains the combined 16 and 17 mutations.
- 28:: Glutamic acid 129 was substituted with glycine using the primer GCCAGATACCCGCTCTGG.
The corresponding gene was modified in this way:
the sequence 990-AGCGAATATCT-1000 was changed into 990-AGCGGGTATCT-1000.
- 29:: Arginine 135 was substituted with glutamic acid using the primer GCGGAATGTCCCGGTGTG.
The corresponding gene was modified in this way:
the sequence 1010-GCGCATTCCGC-1020 was changed into 1010-GGACATTCCGC-1020.
- 31:: Threonine 159 was substituted with lysine using the primer TACTCCGTTTTCGTGGTC.
The corresponding gene was modified in this way:
1070-GCATCACCGGCGAGACCACGACCACGGAGTA-1090 was changed into 1070-GCATCACCGGCGAGACCACGAAAACGGAGTA-1090.
- 26:: Tyrosine 111 was substituted with glycine.
Furthermore, owing to a partial duplication of a primer fragment, the insertion of the Asp Thr Gly and Gly amino acids occurred in position 113 using the primer CGCCACCAGTGTCGACGTATTCGA. The corresponding gene was modified in this way.
930-GTCGACACTTATGGCGACAAT-950
930-GTCGACACTGGTGGCGACACTGGTGGCGACAAT-950.

Said oligonucleotides have been used as primers for DNA polymerase which transcribes all the nucleotide sequence of the vector incorporating the mutations present in the primer.

The vectors containing the S1 gene with the desired modifications were isolated by hybridization using as a probe the primer itself.

The exact nucleotide sequences of the modified genes were then confirmed by the technique of Sanger F. *et al*. (PNAS 74, 5463, 1977).

The vectors containing the modified genes were then digested with the restriction enzymes EcoRI and HindIII and the DNA fragments containing the gene coding for the modified S1 were cloned in an expression plasmid selected from those known in the art.

Said hybrid plasmids have been employed to transform a host microorganism selected from *E. coli, Bacillus subtilis* and yeasts.

In particular, according to the present invention, the plasmid PEx34 (Center for Molecular Biology, Heidelberg, Federal Republic of Germany) and the microorganism *E. coli* K12-ΔH1-Δtrp (Remant, E. *et al*. Gene, 15, 81-93, 1981) were employed.

The transformed microorganisms were then cultivated in a liquid culture medium in the presence of carbon, nitrogen and mineral salt sources, at a temperature between 30°C and 45°C for a period of from 20 minutes to 5 hours.

At the end of the period, the cells were recovered from the culture medium by centrifuging and were lysed using generally known techniques.

The cellular lysates containing the proteins were then analysed to determine the enzymatic activity thereof. The results, reported in the following Example 3, show that a good reduction (5-80%) of the ADP-ribosylation activity, and therefore toxicity, was obtained by substituting amino acids in the S1 sequence at the 109 (17) and 124 (16) positions, either separately or in combination, and the amino acid in 121 position (27).

A complete loss of the S1 subunit enzymatic activity has been observed by substituting amino acids at the 8 and 9 (41) , 50 and 53 (22) and 129 (28) positions.

Furthermore, said subunits are able to induce *in vivo* specific antibodies and to react (subunit 28) with anti-PT protective monoclonal antibodies.

Polypeptides containing said modified subunits, therefore, are suitable for the preparation of an antipertussis vaccine.

Preferred are polypeptides containing in addition to the modified S1 subunit at least one of the S2, S3, S4 and S5 PT subunits.

Particularly, preferred are polypeptides having said S2, S3, S4 and S5 subunits with the same arrangement and configuration presented by the pertussis toxin.

Said preferred polypeptides may be prepared by modifying the gene coding for S1 contained in the PT operon and constructing plasmids, comprising the whole operon with the modified S1 genes or regions thereof, which essentially code for a polypeptide containing the modified S1 subunit and one or more of the S2, S3, S4 and S5 subunits.

According to the present invention, the plasmids PTE 255-22, PTE 255-28 and PTE 255-41, containing the gene which codes for the S1 modified subunits 22, 28 and 41 respectively, were deposited as *E. coli* (PTE 255-22)*, E. coli* (PTE 255-28) and *E. coli* (PTE 255-41) at the American Type Culture Center and designated as ATCC 67542, ATCC 67543 and ATCC 67544.

The following experimental examples are illustrative and non limiting of the invention.

### Example 1

### Identification of the S1 subunit region correlated to the ADP-ribosylation activity

### A. Construction of the hybrid plasmids containing the gene coding for modified S1 by deletion of the 3' terminal part

10 »g of the PTE 255 plasmid were suspended in 100 »l of buffer solution (50 mM Tris-HCl, pH 7.4, 10 mM MgCl₂, 100 mM NaCl) and digested at 37°C for two hours with 30 units (U) of XbaI (BRL) restriction enzyme and then aliquots of 10 »l of the digestion mixture were treated with 3U of one of the following enzymes: NcoI, BalI, NruI, SalI and SphI at 37°C for two more hours.

The DNA mixtures so digested containing the 75 base pairs (bp) XbaI-NcoI, 377 bp XbaI-BalI, 165 bp XbaI-NruI, 355 bp XbaI-SalI and 503 bp XbaI-SphI fragments respectively, were combined with 3 U of Klenow polymerase and with 2 »l of a solution containing 50 mM of each of the following deoxynucleotides: dATP, dTTP, dCTP and dGTP, to repair the molecule ends.

The mixtures were maintained at ambient temperature (20-25°C) for 15 minutes and at 65°C for an additional 30 minutes in such a way as to inactivate the polymerase enzyme.

At the end of said period, the mixtures are diluted to 200 »l with ligase buffer (66 mM Tris-HCl, pH 7.6, 1 mM ATP, 10 mM MgC1₂, 10 mM Dithiothreitol) and were maintained at 15°C for one night in the presence of one unit of T4 DNA ligase so that the DNA molecules which lost the above mentioned fragments were linked to each other again. The ligase mixtures were then employed to transform K12- Hl trp *E. coli* cells prepared by a treatment with 50 mM CaC1₂ (Mandel M. and Higa (1970) I. Mol. Biol. 53, 154).

The transformants were selected by plaguing the cells on LB agar (10g/l Bacto Tryptone (DIFCO), 5 g/l Bacto Yeast extract (DIFCO) 5 g/l NaCl) medium containing 30 »g/ml ampicillin and incubating the plates at 30°C for 18 hours. The recombinant plasmids were analysed in order to verify the exact nucleotide sequence.

The following hybrid plasmids were identified:
PTE NCO: in which the S1 gene lacks a part coding for the carboxy-terminal sequence of the S1 subunit between amino acids 211 and 255.

PTE NRU: where the S1 gene lacks a part coding for the carboxy-terminal sequence of the S1 subunit between amino acids 180 and 255.

PTE BAL: where the S1 gene is lacking a part which codes for the carboxy-terminal sequence of the S1 subunit between amino acids 124 and 255.

PTE SAL: in which the S1 gene is lacking a part coding for the carboxy-terminal sequence of the S1 subunit between amino acids 110 and 255.

PTE SPH: in which the S1 gene is lacking a part coding for the carboxy-terminal sequence of the S1 subunit between amino acids 68 and 255.

### B. Construction of hybrid plasmids containing the gene coding for modified S1 by deletion of the 5' terminal part

3 probes (10 »g) of the PTE 255 plasmid were digested in 100 »l of a buffer solution (50 mM Tris-HCL, pH 7.4, 10 mM MgCl₂, 50 mM NaCl) at 37°C for 3 hours, with 30 U of each of the following restriction enzymes:
SphI, SalI and BalI respectively.
3 U of Klenow polymerase enzyme were then added to each solution together with 2 »l of a solution containing 50 mM of each of the following deoxynucleotides: dATP, dTTP, dCTP and dGTP and after 15 minutes at 20-25°C the enzyme was inactivated at 65°C for 30 minutes.

30 U of HindIII restriction enzyme were then added to each solution and the resulting mixture were maintained at 37°C for 3 hours and then loaded on 1.5% agarose gel and subjected to electrophoresis at 70 Volts for 3.5 hours.

In this way, two bands are separated for each mixture, one containing the deletion part of S1 and the other containing the PeX-34 plasmid and part of S1.

The 520 bp sph-Hind III, 372 bp SalI-Hind III and 394 bp BalI-HindIII fragments were then electroeluted by the Maniatis method (Molecular Cloning: A Laboratory Manual, Cold Spring Harbor, 1982). 100 ng of each of said fragments were then linked, in 30 »l of ligase mixture in the presence of 1 U T4 DNA ligase, with the plasmid Pex-34 previously digested with the BamHI restriction enzyme, treated with polymerase enzyme and a solution of deoxynucleotides and then digested with the HindIII restriction enzyme.

The ligase mixtures were successively employed to transform *E. coli* K12, ΔH1, Δtrp cells and the transformants were selected on LB agar medium containing Ampicillin as reported in Section A.

Among the plasmids extracted from the positive clones, those containing the cloned fragments in a proper reading frame were identified by western-blot with pertussis anti-toxin antibodies.

Said plasmids, labeled with the abbreviations PTE SPH/HIND, PTE 255/SAL and PTE 255/BAL were lacking the S1 gene sequences which code for amino-terminal parts of the subunit between the amino acids: 1-67, 1-109 and 1-123 respectively.

### C. Construction of hybrid plasmids containing the gene coding for modified S1 by deletion of 3' and 5' terminal parts

2 samples (10 »g) of the plasmid PTE NCO obtained as illustrated in Section A) were digested in 100 »l of 50 mM Tris-HCl, pH 7.4, 10 mM MgCl₂, 50 mM NaCl solution, with 30 U of BstN1 (BRL) and 30 U of BalI (BRL) respectively, at 37°C for 3 hours.

The digestion mixtures were then treated at 20-25°C for 15 minutes with 3 U of klenow polymerase enzyme in the presence of 2 mM of dATP, dGTP, dCTP and dTTP to complete the terminal portions and, after inactivation of the enzyme at 65°C for 30 minutes, the DNA was again cut with 30 U of HindIII restriction enzyme at 37°C for 3 hours.

The digestion mixtures were loaded on 1.5% agarose gel and eluted subjected to electrophoresis at 70 Volts for 3.5 hours, with the 527 bp BstN1-HindIII and the 279 bp Ba1I-HindIII fragments being electro-eluted as reported above.

100 ng of said fragments were subsequently linked to the plasmid PeX-34, previously treated as reported in Section B in a ligase mixture in the presence of 7 U T4 DNA ligase at 15°C for 18 hours. The transformation of the *E. coli* cells and the selection of the transformants was then performed as illustrated above. The fragments inserted in the right reading frame were identified among the recombinant plasmids extracted from the positive clones.

Said plasmids, labeled with the abbreviation PTE 34A and PTE NCO/BAL contain respectively the S1 gene without the sequences coding for the S1 subunit part between amino acids 1-124 and 211-255.

### D. Construction of PTE 16-A and 18-A plasmids

10 »g of the PTE 255 plasmid were digested in 100 »l of 100 mM Tris-HCl, 50 mM NaCl, 10 mM MgSO₄ buffer with 30 U of EcoRI and then with 1U of Bal31 (BRL) in 10 mM CaC1₂, 10 mM MgCl₂, 0.2M M NaCl, 20 mM Tris-HCl, pH 8, 1mM EDTA at 37°C. Mixture aliquots were withdrawn after 1, 3, 5 and 10 minutes and the deletion fragments at the 5' terminal were then cut with HindIII, purified by gel electrophoresis and, after elution, linked to the Pex-34 plasmid as reported above. The ligase mixture were then employed to transform the *E*. *coli* cells and the transformants were selected as reported in the preceding steps.

The plasmids containing the S1 gene fragments inserted in the right reading frame, determined by their nucleotide sequence analysis, were isolated from the plasmids extracted from the positive clones.

The plasmids containing the S1 gene without the sequence which codes for the S1 amino-terminal part were selected from the plasmids so obtained.

In particular, the PTE 16-A plasmid lacked the nucleotides coding for the first 10 amino acids and therefore coded for a protein containing amino acids 11-235, whereas the PTE 18-A plasmid coded for a protein containing amino acids 149-235.

### Example 2

### Expression of the modified S1 subunits and determination of the ADP-ribosylation activity thereof

A. K12, ΔH1, Δtrp *E. coli* cells, transformed with the plasmids prepared as reported in Example 1, were cultivated in 20 ml of liquid LB medium under smooth mixing at 30°C for one night.

10 ml of each culture was employed to inoculate 400 ml of LB medium and cultivated at 30°C for 2 hours and at 42°C for 2.5 hours.

At the end of said period, the cultures were centrifuged at 10,000 r.p.m. for 15 minutes at 4°C, the supernatants discarded, the cells recovered and then resuspended in 3.2 ml of 2.5% sucrose, 10 mM Tris-HCl (pH 8.0), 1 mM EDTA solution.

0.1 ml of a lysozyme solution (40 mg/ml) and 0.8 ml of 0.5 M EDTA were added to the solutions which were then incubated at 37°C for 30 minutes. 8 ml of a lysis buffer (1% Triton-X 100, 50 mM Tris-HCl, pH 6.0, 63 mM EDTA) was then added to each solution which was maintained at 0°C for 15 minutes and at 37°C for 30 minutes.

After a 1 minute sonication, the mixtures containing the lysed cells and their contents were centrifuged at 10,000 r.p.m. for 10 minutes, the supernatants were discarded and the precipitates resuspended in 5 ml of 1M urea and maintained at 37°C for 30 minutes.

The mixtures were again centrifuged and the precipitates or cellular contents were recovered and dissolved in 5 ml of phosphate buffered saline (PBS) and frozen at -20°C.

### B. Analysis of the ADP-ribosylating activity

The solutions containing the cellular contents were centrifuged and the precipitates resuspended in 100 »l of 8 M urea before performing the ADP-ribosylation test.

The ADP-ribosylation test was performed according to the technique described by Manning *et al*. (1984). (J. Biol. Chem. 259, 749-756).

10 »l of each solution was preincubated with a 20 »l solution of 100 mM of Dithiothreitol at 20-25°C for 30 minutes and then added to 10 »l of ox retina homogenate (ROS) , 80 »l of water, 5 »l Tris-HCl (pH 7.5), 1 »l of an 100 mM ATP solution, 1 »l of 10 mM GTP solution, 10 ml of thymidine and 1 »l (1 nCi) ³²PNAD.

The mixtures were then reacted at ambient temperature (20-25°C) for 30 minutes and, after centrifugation, the residues containing the ROS were recovered and dissolved in 30 »l of sodium dodecyl sulphate (SDS) buffer and loaded on a 12.5% polyacrylamide gel. After electrophoresis at 25 mA for 4 hours, the gels were vacuum-dried at a temperature of 80°C and then subjected to autoradiography. The radioactive bands were separated from the gel, suspended in 5 ml of scintillation liquid (Econofluor, NEN) and counted in a beta counter.

In this way, the ADP-ribosylation of the modified proteins was quantitatively determined.

The results obtained are reported in the following Table I:

**TABLE I**

| Plasmids containing the modified S1 gene | ADP-ribosylation activity of the modified S1 (%) |
|---|---|
| PTE NCO | 100 |
| PTE NRU | 60 |
| PTE BAL | - |
| PTE SAL | - |
| PTE SPH | - |
| PTE 16-A | - |
| PTE 34-A | - |
| SPH/HIND | - |
| 255/BAL | - |
| 255/SAL | - |
| NCO/BAL | - |
| 18-A | - |

Its clearly apparent from what is disclosed in Table I, that the sequences following the Nru site (179 position) are not necessary, as opposed to the 5' terminal sequences, for the ADP-ribosylation activity of the S1 subunit.

### Example 3

### Identification and mutation of the active sites of the 1-180 region of the S1 subunit

10 »g of the PTE 255 plasmid was suspended in 100 »l of 10 mM Tris-HCl, pH 7.5, 50 mM NaCl, 10 mM MgCl₂ buffer and digested with 30 U of each of the EcoRI and Hind-III enzymes at 37°C for 3 hours.

The digestion mixture was then loaded on 1.3% agarose gel and eluted at 80 mA for 3 hours.

In this way, two bands were separated; one of 3,500 bp containing the vector and the other of 600 bp containing the gene which codes for the S1 subunit.

The 600 bp band was then electro-eluted and 0.2 »g of the fragment and 0.3 ng of the Bluescript SK (Stratagene, San Diego, CA) plasmid, previously digested with the EcoRI and HindIII restriction enzymes were suspended in 20 »l of buffer solution (66 mM Tris-HCl, pH 7.5, 1 mM ATP, 10 mM MgCl₂, 10 mM Dithiothreitol) and linked together in the presence of 1U T₄ DNA ligase at 15°C for 18 hours.

The ligase mixture was then employed to transform competent JM 101 *E. coli* cells and the transformants were selected on plates of LB agar including 100 »g/ml Ampicillin, 20 »g/ml IPTG (isopropyl-B-D-thiogalactopyranoside) and 20 »g/ml X-Gal (5-bromo-4-chloro-3-indolyl-D-galactopyranoside).

The plates were incubated at 37°C in a thermostatic chamber for 18 hours. The white colonies containing the hybrid plasmid comprising the Bluescript SK vector and the 600 bp DNA fragment were used to isolate the single-stranded DNA of the cloned fragment, as follows.

The white colonies were cultivated in 1.5 ml LB liquid medium in order to reach an optical density, (OD) at 590 mm of about 0.15.

10 »l of an F1 phage (Stratagene San Diego, CA) suspension in LB (5x10¹² phages/ml) was subsequently added to the cultures and the resulting solutions were maintained at 37°C for 6-8 hours.

At the end of said period, the cells were separated from the culture medium by centrifugation and the supernatant recovered. 20% polyethyleneglycol (PEG) and 2.5 mM NaCl were added to 1 ml of said supernatant to precipitate the phages.

After 15 minutes at ambient temperature (20-25°C), the mixture was centrifuged at 12,000 r.p.m. for 5 minutes in an Eppendorf centrifuge at 20°C and the phages so recovered resuspended in 100 »l TE (10 mM Tris-HCl, pH 7.5, 1 mM EDTA) buffer.

The solution was then extracted once with one volume of water-saturated phenol, twice with ethyl ether and finally, the single stranded helix DNA was precipitated by adding to the aqueous phase 250 »l of ethanol and 10 »l of 3 M ammonium acetate. The DNA was separated from the mixture by centrifugation, resuspended in 20 »l of TE buffer and employed for site-directed mutagenesis (Zoller *et al*. DNA, 3, 479-488, 1984).

To this end, oligonucleotides, in which the bases coding for at least one of the desired amino acids were modified in order to code for another amino acid, were synthesized by means of a 1 Plus DNA synthesizer System (Beckman) automatic system.

Said oligonucleotides, complementary to the sequence present in the single-stranded DNA cloned in the Bluescript SK plasmid, were used as primers for the DNA polymerase which transcribes the whole Bluescript nucleotide sequence incorporating the mutations present in the primer.

2 »l of 10 mM ATP, 2 »l of 10 X Kinase buffer (550) mM Tris-HCl, pH 8.0, 100 mM MgCl₂), 1 »l of 100 mM Dithiothreitol (DTT) and 5 U of polynucleotide kinase (Boehringer) were added to 3 mM of the synthetic oligonucleotide and the final volume was brought up to 20 »l.

The mixture was incubated at 37°C for 30 minutes and the enzyme inactivated at 70°C for 10 minutes.

1 »g of the single strand used as a template, 1 »l of 1 mM Tris-HCl, pH 8.0, and 10 mM MgCl₂ in 1 volume of 10 X Kinase buffer were added to 2 »l of the primer.

The mixture was maintained at 80°C for about 3 minutes and then at ambient temperature for about 1 hour. 10 »l of 1 mM Tris-CHl, pH 8.0, 10 mM MgCl₂ buffer, 0.05 mM ATP, 1 mM DTT, 0.5 mM of the four deoxynucleotides, 1 U of T4 DNA ligase and 2.5 U of I DNA polymerase (Klenow Fragment) were subsequently added.

The mixture was incubated at 15°C for one night and then used to transform JM 101 *E. coli* cells as illustrated above.

The plasmids containing the mutated S1 gene were then identified by hybridization using as a probe the primer used for the mutagenesis, labelled with ³²P. The nitrocellulose filters containing the transformed cultures were hybridized in 6xSSC (1xSSC=0.015 M NaCl, 0.015M trisodium citrate, pH7), 10xDenhardts (1% BSA, 1% Ficoll, 1% Polyvinylpyrrolidone) and 0.2% Sodium-dodecylsulphate (SDS) at 20-25°C for 18 hours and then washed for 2 hours in 6xSSC at the following temperatures: (45°C) 25 and 26 mutants; (48°C) 28, 22 and 29 mutants; (54°C) 27; (46°C) 31 and 41 mutants.

The mutations were confirmed by analysis of the nucleotide sequence of the gene according to the method of Sanger, F. *et al*. (PNAS 74, 5463, 1977).

As reported above, plasmids containing the gene coding for S1 modified were prepared as follows:
- 41:: 8 Tyrosine and 9 arginine were substituted with Aspartic acid and Glycine respectively, using the GTCATAGCCGTCTACGGT primer.
The corresponding gene was thus:
620-CGCCACCGTATACCGCTATGACTCCCGCCCG-650
620-CGCCACCGTAGACGGCTATGACTCCCGCCCG-650
- 22:: 50 phenylalanine and 53 threonine were substituted with glutamic acid and isoleucine respectively, using the TGGAGACGTCAGCGCTGT primer.
The corresponding gene was modified thus:
The 750-AGCGCTTTCGTCTCCACCAGC-770 sequence was changed into 750-AGCGCTGACGTCTCCATCAGC-770.
- 25:: 99 glycine was substituted with glutamic acid using the CTGGCGGCTTCGTAGAAA primer.
The corresponding gene was modified thus:
the 910-TACGGCGCCGC-920 sequence was changed into 910-TACGAAGCCGC-920.
- 17:: 109 aspartic acid was substituted with glycine using the CTGGTAGGTGTCCAGCGCGCC primer.
The corresponding gene was modified thus;
the 930-GTCGACACTTA-940 sequence was changed into 930-GTCGGCACTTA-940.
- 27;: 121 glycine was substituted by glutamic acid using the GCCAGCGCTTCGGCGAGG primer.
The corresponding gene was modified thus:
the 956-GCCGGCGCGCT-966 sequence was changed into 956-GCCGAAGCGCT-966.
- 16:: Alanine in position 124 was substituted with aspartic acid using the GCCATAAGTGCCGACGTATTC primer. The corresponding gene was modified thus:
the 976-TGGCCACCTAC-984 sequence was changed into 976-TGGACACCTAC-986.
- 1716:: contains the combined 16 and 17 mutations.
- 28:: 129 glutamic acid was substituted with glycine using the GCCAGATACCCGCTCTGG primer.
The corresponding gene was modified thus:
the 990-AGCGAATATCT-1000 sequence was changed into 990-AGCGGGTATCT-1000.
- 29:: 135 arginine was substituted with glutamic acid using the GCGGAATGTCCCGGTGTG primer.
The corresponding gene was modified thus:
the 1010-GCGCATTCCGC-1020 sequence was changed into 1010-GGACATTCCGC-1020.
- 31:: 159 threonine was substituted with lysine using the TACTCCGTTTTCGTGGTC primer.
The corresponding gene was modified thus:
1070-GCATCACCGGCGAGACCACGACCACGGAGTA-1090 was changed into 1070-GCATCACCGGCGAGACCACGAAAACGGAGTA-1090.
- 26:: 111 tyrosine was substituted with glycine. Furthermore, owing to a partial duplication of a primer fragment, the insertion of the Asp Thr Gly and Gly amino acids occurred in the position 113 using the CGCCACCAGTGTCGACGTATTCGA primer. The corresponding gene was modified thus:
930-GTCGACACTTATGGCGACAAT-950
930-GTCGACACTGGTGGCGACACTGGTGGCGACAAT-950.

The plasmids containing the S1 gene were digested again with the EcoRI and HindIII restriction enzymes and the DNA fragment containing the above mentioned mutations separated from the digestion mixture by gel electrophoresis, electroeluted and cloned in the PEx-34B vector in a ligase mixture as reported above.

The ligase mixtures were used to transform suitable K12, ΔH1, Δtrp *E. coli* cells and the transformants isolated on LB agar medium containing 30 »g/ml of Ampicillin at 30°C.

The positive clones containing the mutated plasmids were then cultivated in LB liquid medium as reported in Example 2 and, after cellular lysis, the ADP-ribosylating activity of the S1 subunits so obtained was determined.

The results are reported in the following Table II:

**Table II**

| Mutant subunits | ADP-ribosylating activity of the mutated subunits (%) |
|---|---|
| 41 | 0 |
| 22 | 0 |
| 25 | 100 |
| 17 | 46 |
| 26 | 150 |
| 27 | 43 |
| 16 | 50 |
| 1617 | 23 |
| 28 | 0 |
| 29 | 92 |
| 31 | 100 |
| BppB | 100 |

BppB and BBp are S1 hybrids containing respectively the gene of *B. pertussis* up to SalI and the remaining section of B. *bronchiseptica* and vice versa.

The results reported above the mutant 28, in which the substitution of only one amino acid resulted in the complete loss of the enzymatic activity, seem particularly interesting.

## Claims

1. An immunologically active polypeptide with no or reduced toxicity, useful for the preparation of an antipertussis vaccine, wherein said polypeptide contains the S1 subunit of the pertussis toxin modified by the substitution of an amino acid with another capable of destroying or reducing the toxicity of S1 without altering the immunological properties thereof wherein at least glutamic acid (129) is substituted with another amino acid.

2. A polypeptide according to claim 1, containing additionally at least one of the S2, S3, S4 and S5 subunits of the pertussis toxin.

3. A polypeptide according to claim 2, wherein the S2, S3, S4 and S5 subunits have the same arrangement as that present in the natural pertussis toxin.

4. A method for the preparation of a polypeptide according to any one of the preceding claims comprising:
a) modifying by direct mutagenesis the gene coding for the S1 subunit of the pertussis toxin by substituting in one or more sites of the DNA molecule the base sequence for an amino acid with a base sequence which codes for a different amino acid wherein at least the base sequence which codes for glutamic acid in position 129 is substituted for a base sequence which codes for a different amino acid;
b) constructing a hybrid plasmid linking a cloning vector with the DNA fragment containing the gene coding for the modified S1 subunit;
c) transforming a host microorganism with a hybrid plasmid obtained as in b);
d) cultivating a transformed microorganism in a suitable culture medium in the presence of a carbon, nitrogen and mineral salts sources and finally,
e) recovering the polypeptide containing the modified subunit from the culture medium or from the cells.

5. A method according to claim 4, wherein in the step a) the gene coding for the S1 subunit is contained in the pertussis toxin operon.

6. A method according to claim 4, wherein in the step b) the DNA fragment contains additionally at least one gene coding for the S2, S3, S4 or S5 subunits of the pertussis toxin.

7. A method according to claim 6, wherein said genes are grouped in the operon which codes for the pertussis toxin.

8. A method according to claim 4, wherein in the step c) the microorganism is chosen in the group comprising, Escherichia coli, bacillus and yeasts.

9. A method according to claim 8, wherein the microorganism is Escherichia coli (E.coli).

10. E.coli (PTE 255-28), ATCC 67543, transformed microorganisms.

11. An antipertussis vaccine containing a therapeutically effective quantity of at least one polypeptide according to any one of claims 1 to 3.

12. A gene coding for an immunologically active polypeptide as defined in any one of claims 1 to 3.

13. A hybrid plasmid containing the gene as defined in claim 12.

14. A microorganism transformed with a hybrid plasmid as defined in claim 13.

## Patentansprüche

1. Immunologisch aktives Polypeptid mit keiner oder reduzierter Toxizität, verwendbar für die Herstellung eines Antipertussis-Impfstoffes, wobei das Polypeptid die S1-Untereinheit des Pertussis-Toxins enthält, modifiziert durch die Substitution einer Aminosäure durch eine andere, die fähig ist, die Toxizität von S1 zu zerstören oder zu reduzieren, ohne dessen immunologische Eigenschaften zu verändern, wobei mindestens Glutaminsäure (129) durch eine andere Aminosäure substituiert wird.

2. Polypeptid nach Anspruch 1, zusätzlich enthaltend mindestens eine der Untereinheiten S2, S3, S4 und S5 des Pertussis-Toxins.

3. Polypeptid nach Anspruch 2, wobei die Untereinheiten S2, S3, S4 und S5 dieselbe Anordnung wie die im natürlichen Pertussis-Toxin vorliegende haben.

4. Verfahren zur Herstellung eines Polypeptids nach einem der vorangegangenen Patentansprüche, umfassend:
(a) Modifizierung des für die S1-Untereinheit des Pertussis-Toxins codierenden Gens durch direkte Mutagenese durch Substitution der Basen-Sequenz für eine Aminosäure an einer oder mehreren Stellen des DNA-Moleküls durch eine Basensequenz, die eine andere Aminosäure codiert, wobei mindestens die Basensequenz, die für Glutaminsäure in Position 129 codiert, durch eine Basensequenz substituiert wird, die für eine andere Aminosäure codiert;
(b) Konstruktion eines Hybridplasmids durch Ligierung eines Clonierungsvektors mit dem DNA-Fragment, das das Gen für die modifizierte S1-Untereinheit enthält;
(c) Transformation eines Wirtsmikroorganismus mit einem gemäß (b) erhaltenen Hybridplasmid;
(d) Züchtung eines transformierten Mikroorganismus in einem geeigneten Kulturmedium in Gegenwart einer Kohlenstoff-, Stickstoff- und Mineralsalz-Quelle und, schließlich
(e) Gewinnung des Polypeptids, enthaltend die modifizierte Untereinheit aus dem Kulturmedium oder aus den Zellen.

5. Verfahren nach Anspruch 4, wobei in Schritt (a) das für die S1-Untereinheit codierende Gen im Pertussis-Toxin-Operon enthalten ist.

6. Verfahren nach Anspruch 4, wobei in Schritt (b) das DNA-Fragment zusätzlich mindestens ein Gen enthält, das für die Untereinheiten S2, S3, S4 oder S5 des Pertussis-Toxins codiert.

7. Verfahren nach Anspruch 6, wobei die Gene in dem Operon angeordnet sind, das für das Pertussis-Toxin codiert.

8. Verfahren nach Anspruch 4, wobei im Schritt (c) der Mikroorganismus aus der Gruppe ausgewählt wird, die Escherichia coli, Bacillus und Hefen umfaßt.

9. Verfahren nach Anspruch 8, wobei der Mikroorganismus Escherichia coli (E. coli) ist.

10. E. coli (PTE 255-28), ATCC 67543, transformierte Mikroorganismen.

11. Antipertussis-Impfstoff, enthaltend eine therapeutisch wirksame Menge mindestens eines Polypeptids nach einem der Ansprüche 1 bis 3.

12. Gen, das ein immunologisch aktives Polypeptid wie in einem der Ansprüche 1 bis 3 definiert codiert.

13. Hybridplasmid, enthaltend das wie in Anspruch 12 definierte Gen.

14. Mikroorganismus, transformiert mit einem Hybridplasmid wie in Anspruch 13 definiert.

## Revendications

1. Polypeptide à activité immunologique ayant une toxicité nulle ou réduite, utile pour la préparation d'un vaccin anticoquelucheux (antipertussis), dans lequel ledit polypeptide contient la sous-unité S1 de la toxine de la coqueluche modifiée par la substitution d'un acide aminé par un autre apte à annihiler ou réduire la toxicité de S1 sans modifier les propriétés immunologiques de celle-ci, au moins l'acide glutamique (129) étant substitué par un autre acide aminé.

2. Polypeptide selon la revendication 1, contenant en outre au moins l'une des sous-unités S2, S3, S4 et S5 de la toxine de la coqueluche.

3. Polypeptide selon la revendication 2, dans lequel les sous-unités S2, S3, S4 et S5 ont le même arrangement que celles présentes dans la toxine naturelle de la coqueluche.

4. Procédé pour la préparation d'un polypeptide selon l'une quelconque des revendications précédentes, comprenant:
a) la modification par mutagénèse directe du gène codant pour la sous-unité S1 de la toxine de la coqueluche par substitution sur un ou plusieurs sites de la molécule d'ADN de la séquence de bases pour un acide aminé par une séquence de base codant pour un acide aminé différent, et dans lequel au moins la séquence de bases qui code pour l'acide glutamique en position 129 est substitué par une séquence de bases codant pour un acide aminé différent ;
b) la construction d'un plasmide hybride liant un vecteur de clonage avec le fragment d'ADN contenant le gène codant pour la sous-unité S1 modifiée;
c) la transformation d'un microorganisme hôte par un plasmide hybride obtenu comme dans b);
d) la culture d'un microorganisme transformé dans un milieu de culture approprié en présence de sources de carbone, d'azote et de sels minéraux, et finalement
e) la récupération du peptide contenant la sous-unité modifiée à partir du milieu de culture ou des cellules.

5. Procédé selon la revendication 4, dans lequel dans l'étape a) le gène codant pour la sous-unité S1 est contenu dans l'opéron de la toxine de la coqueluche.

6. Procédé selon la revendication 4, dans lequel dans l'étape b) le fragment d'ADN contient en outre au moins un gène codant pour les sous-unités S2, S3, S4 ou S5 de la toxine de la coqueluche.

7. Procédé selon la revendication 6, dans lequel les dits gènes sont groupés dans l'opéron qui code pour la toxine de la coqueluche.

8. Procédé selon la revendication 4, dans lequel dans l'étape c) le microorganisme est choisi dans le groupe comprenant Escherichia coli, des bacilles et des levures.

9. Procédé selon la revendication 8, dans lequel le microorganisme est Escherichia coli (E. coli).

10. Microorganismes transformés d'E. coli (PTE 255-28), ATCC 67543.

11. Vaccin de la coqueluche contenant une quantité thérapeutiquement efficace d'au moins un polypeptide selon l'une quelconque des revendications 1 à 3.

12. Gène codant pour un polypeptide à activité immunologique tel que défini dans l'une quelconque des revendications 1 à 3.

13. Plasmide hybride contenant le gène tel que défini dans la revendication 12.

14. Microorganisme transformé avec un plasmide hybride tel que défini dans la revendication 13.
